# EUROPEAN PATENT APPLICATION

(11) **EP 1 894 936 A1**
(43) Date of publication of application: **05.03.2008**
(21) Application number: 07115315.9
(22) Date of filing: 30.08.2007
(51) Int. Cl.: C07F 7/18, A61Q 17/04, A61P 17/18

(54) **Organosilyl-Benzo-triazole derivatives, processes for their production and their use as UV protecting agents**

(30) Priority: 31.08.2006 EP 06018235
(71) Applicant: ISDIN, S.A., 08006 Barcelona (ES)
(72) Inventor: Trullas, Carles, 08006, Barcelona (ES); Corbera, Jordi, 08041, Barcelona (ES); Jiménez, Oscar, 08006, Barcelona (ES); Gorchs Capa, Olga, 08006, Barcelona (ES); Novac, Lajos, Gellért ter 4 1111, Budapest (HU)
(74) Representative: Barlocci, Anna

(57) **Abstract**

The present invention refers to new benzotriazole derivatives, which for their physicochemical properties can be used as UV protection agents. In addition, they can be used to manufacture cosmetic, dermatological, veterinarian as well as pharmaceutical formulations, which protect the skin, lips, nails and hair against UV radiation.

## Description

### Field of the invention

The present invention relates to the field of dermatology, cosmetics and pharmaceuticals. In particular, the present invention refers to new benzotriazole derivatives, which for their physicochemical properties are useful as UV protecting agents. In addition, they are particularly suited to manufacture cosmetic, dermatological, veterinarian as well as pharmaceutical formulations, which protect the skin, lips, nails and hair against UV radiation.

### Background art

The sun light and especially the ultraviolet radiation can in certain circumstances provoke damaging effects on the skin and can even create pathological situation such as e.g. dermatosis.

The principal cause for these pathologies is the ultraviolet radiation, whose energy is inversely proportionate to its wavelength. Therefore, the shorter the wavelength the more energy it has. UV radiation can be classified in UV-C, UV-B and UV-A, of which the UV-C is the most damaging, even if it is absorbed by the ozone layer.

Before UV-A and UV-B radiation could cause damage the skin is equipped with different natural protection systems which could absorb or deflect the radiation, like melanin etc.

In this area and for the cause of reducing the effects of the solar radiation, solar filters and/or sunscreens are actually used. These solar filters and/or sunscreens are compounds which are applied to the skin, lips, nails or hair and which can be found in cosmetic, dermatological and pharmaceutical formulations as well as other cosmetic products for the protection against solar radiation, while escaping the decomposition of the active principle or radiation sensible components.

In the last years compounds have been identified by investigation which for their physicochemical properties are quite effective as solar filters and/or sunscreen.

Despite the great diversity of solar filters and/or sunscreen, there is still a need for new compounds that for their physicochemical properties are suitable solar filters and/or sunscreen for the protection against UV radiation, especially showing a simultaneous protection against the UV-A and UV-B radiation, with preferably a proportionately high absorbance in the area of UVA.

### Description of the invention:

Thus, in one of its aspects the present invention relates to benzotriazole derivatives of general formula I, 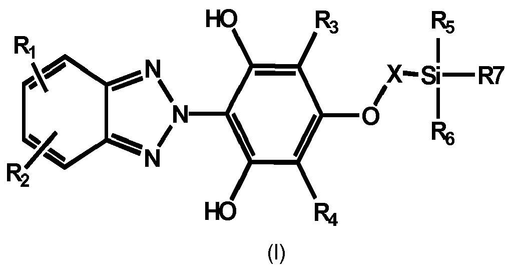 wherein
R₁ and R₂ independently from another are selected from H, OH, SH, F, Cl, Br, I, NH₂; (C₁-C₁₀)alkyl, branched or linear, saturated or unsaturated, substituted or un-substituted; O-(C₁-C₁₀)alkyl, branched or linear, saturated or unsaturated, substituted or un-substituted;
R₃ and R₄ independently from another are selected from H, OH, SH, F, Cl, Br, I, NH₂; (C₁-C₁₀)alkyl, branched or linear, saturated or unsaturated, substituted or un-substituted; O-(C₁-C₁₀)alkyl, branched or linear, saturated or unsaturated, substituted or un-substituted;
R₅, R₆, R₇ independently from another are selected from H; (C₁-C₄)alkyl or O-(C₁-C₄)alkyl, branched or linear, saturated or unsaturated, substituted or un-substituted, an (C₅-C₂₀)aryl group, or O-(C₅-C₂₀)aryl group, substituted or un-substituted; (C₁-C₄)alkyl-(C₅-C₂₀)aryl or O-(C₁-C₄)alkyl-(C₅-C₂₀)aryl, branched or linear, saturated or unsaturated, substituted or un-substituted; or a group of general formula (II) 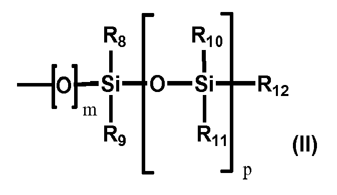 in which
   m=0 or 1;
   p= 0, 1, 2, 3, or 4;
   R₈, Rg, R₁₀, R₁₁ and R₁₂ are identical or different from each other and represent an optionally substituted (C₁-C₆)alkyl; an O-(C₁-C₆)alkyl radical; an optionally substituted (C₅-C₂₀)aryl radical or an -OSi(R₁₃)₃ radical;
R₁₃ represents an (C₁-C₆)alkyl radical, an O-(C₁-C₆)alkyl radical or an optionally substituted (C₅-C₂₀)aryl radical;
X is a connecting alkyl-group with 1, 2, 3, 4, 5 or 6 carbon atoms; branched or linear, saturated or unsaturated, substituted or un-substituted;
wherein the substituents are selected from the group consisting of F, Cl, Br, I, NH₂, SH and OH;
optionally in the form of any of its racemates or tautomers; in the form shown or in form of a salt, especially a physiologically, dermatologically or cosmetically acceptable salt, or in form of a solvate, especially a hydrate.

These compounds are very effective UV-Filters and show a high UV absorption rate and are thus very useful as UV filter agents. Advantageously a high proportion of their absorption is in the UV-A range. In addition, they seem to have advantageous physicochemical properties allowing especially an effective formulation of the compound in a formulation according to the invention. The compounds of the present invention are particularly effective to protect against photodermatosis caused by UV-A radiation.

According to the present invention, the terms "alkyl", "alkenyl" and "alkynyl" embraces both straight and branched hydrocarbons, which can be un-substituted or mono- or polysubstituted. Accordingly, unless defined otherwise, the term "alkyl" encompasses both straight and branched hydrocarbons, which can be un-substituted or mono- or polysubstituted, saturated or unsaturated.

Thus unsaturated alkyl is understood to encompass alkenyl and alkynyl groups, like e.g. -CH=CH-CH₃ or -C=C-CH₃, while saturated alkyl encompasses e.g. -CH₃ and -CH₂-CH₃. In these radicals, (C₁-C₂)alkyl represents C1- or C2-alkyl, (C₁-C₃)alkyl represents C1-, C2- or C3-alkyl, (C₁-C₄)alkyl represents C1-, C2-, C3- or C4-alkyl, (C₁-C₅)alkyl represents C1-, C2-, C3-, C4-, or C5-alkyl, (C₁-C₆)alkyl represents C1-, C2-, C3-, C4-, C5- or C6-alkyl, (C₁-C₇)alkyl represents C1-, C2-, C3-, C4-, C5-, C6- or C7-alkyl, (C₁-C₈)alkyl represents C1-, C2-, C3-, C4-, C5-, C6-, C7- or C8-alkyl, (C₁-C₁₀)alkyl represents C1-, C2-, C3-, C4-, C5-, C6-, C7-, C8-, C9- or C10-alkyl and (C₁-C₁₈)alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In connection with alkyl group - unless defined otherwise - the term substituted in the context of this invention is understood as meaning the replacement of at least one hydrogen radical by F, Cl, Br, I, NH₂, SH or OH, including "polysubstituted" radicals which are being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂.

Specifically in connection with "X" being a connecting alkyl-group with 1, 2, 3, 4, 5 or 6 carbon atoms; branched or linear, saturated or unsaturated, substituted or un-substituted, this signifies that the alkyl group is connecting the oxygen in the ether to the first Si-atom and that the connecting group may in itself be linear or branched, saturated or un-saturated as well as substituted. Examples (which may be further substituted as well as unsaturated) are exemplified below:

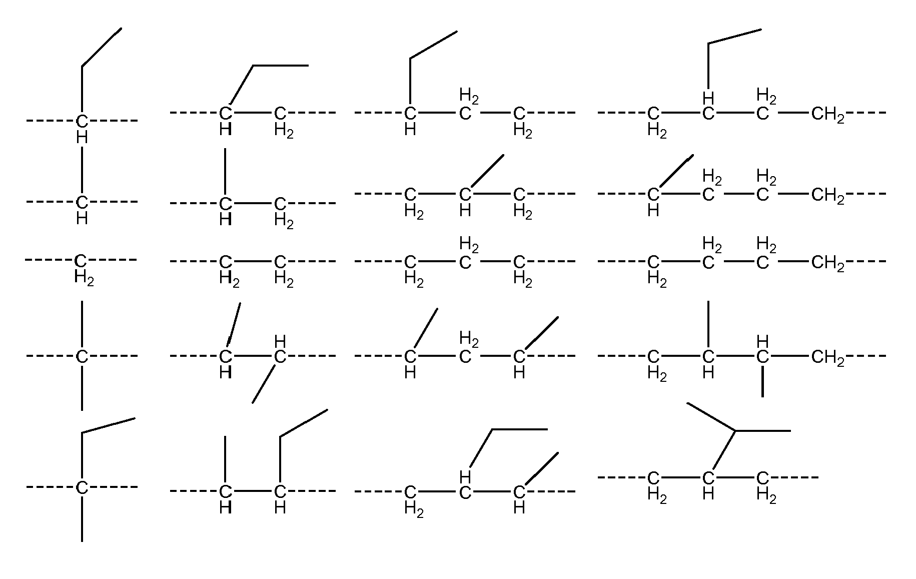

In the context of the present invention, the term "aryl" embraces a radical derived from one of the known carbocyclic from 5 to 20 carbon atoms ring systems with 1-4 rings, wherein each one of the rings forming said ring system is partially unsaturated or aromatic, and is partially or totally fused; being each ring forming part of the ring system optionally substituted by at least one radical selected from the group consisting of: F, Cl, Br, I, OH, NO₂, NH₂, and SH.

Examples of particularly preferred aryl groups include anthracenyl, fluorenyl, phenanthrenyl, phenyl, naphthyl, and the like.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

The term "dermatologically acceptable salt" means in the context of this invention any salt that is dermatologically tolerated (most of the time meaning not being toxic- especially not caused by the counter-ion) if used appropriately for a dermatological treatment especially if used on or applied to humans and/or mammals.

The term "cosmetically acceptable salt" means in the context of this invention any salt that is cosmetically tolerated (most of the time meaning not being toxic- especially not caused by the counter-ion) if used appropriately for a cosmetic treatment especially if used on or applied to humans and/or mammals.

These physiologically/dermatologically/cosmetically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts.

These physiologically/dermatologically/cosmetically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids that are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds that differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵ N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or pro-drugs.

In a preferred embodiment, the benzotriazole derivatives according general formula I 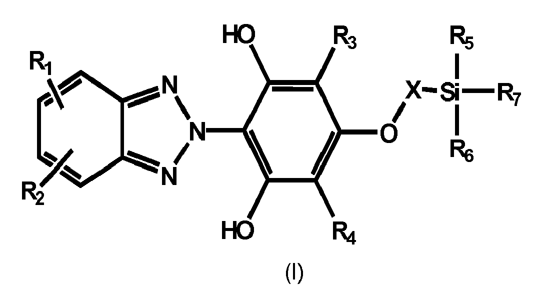 according to the invention are compounds wherein
R₁ and R₂ independently from another are selected from H, OH, SH, F, Cl, Br, I, NH₂; (C₁-C₁₀)alkyl, branched or linear, saturated or unsaturated, substituted or unsubstituted; O-(C₁-C₁₀)alkyl, branched or linear, saturated or unsaturated, substituted or unsubstituted;
R₃ and R₄ independently from another are selected from H, OH, SH, F, Cl, Br, I, NH₂; (C₁-C₁₀)alkyl, branched or linear, saturated or unsaturated, substituted or unsubstituted; O-(C₁-C₁₀)alkyl, branched or linear, saturated or unsaturated, substituted or un-substituted;
R₅, R₆, R₇ independently from another are selected from H; (C₁-C₄)alkyl or O-(C₁-C₄)alkyl, branched or linear, saturated or unsaturated, substituted or un-substituted, an (C₅-C₂₀)aryl group, or O-(C₅-C₂₀)aryl group, substituted or un-substituted; (C₁-C₄)alkyl-(C₅-C₂₀)aryl, branched or linear, saturated or unsaturated, substituted or un-substituted; or a group of general formula (II) 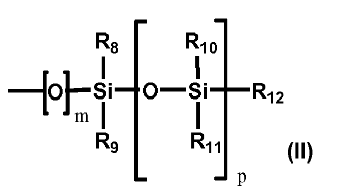 in which
   m= 1;
   p= 0, 1, 2, 3, or 4;
   R₈, R₉, R₁₀, R₁₁ and R₁₂ are identical or different from each other and represent an optionally substituted (C₁-C₆)alkyl radical; an O-(C₁-C₆)alkyl radical; or an -OSi(R₁₃)₃ radical;
R₁₃ represents an (C₁-C₆)alkyl radical, or an O-(C₁-C₆)alkyl radical;
X is a connecting alkyl-group with 1, 2, 3, 4, 5 or 6 carbon atoms; branched or linear, saturated or unsaturated, substituted or un-substituted;
optionally in the form of any of its racemates or tautomers; in the form shown or in form of a salt, especially a physiologically, dermatologically or cosmetically acceptable salt, or in form of a solvate, especially a hydrate.

In another preferred embodiment, the benzotriazole derivatives according general formula I according to the invention are compounds wherein
one or two of the radicals R₅, R₆ or R₇ represent (C₁-C₄)alkyl, branched or linear, saturated or unsaturated, substituted or un-substituted, while the remaining two or one of the radicals R₅, R₆ or R₇ represent a group of general formula (II) as defined above, preferably one of the radicals R₅, R₆ or R₇ represents (C₁-C₄)alkyl, branched or linear, saturated or unsaturated, substituted or un-substituted, while the remaining two radicals R₅, R₆ or R₇ represent a group of general formula (II) as defined above.

In another preferred embodiment, the benzotriazole derivatives according general formula I according to the invention are compounds wherein
R₅, R₆ and R⁷ independently from another are selected from H; (C₁-C₄)alkyl or O-(C₁-C₄)alkyl, branched or linear, saturated or unsaturated, substituted or un-substituted, preferably are O-(C₁-C₄)alkyl, branched or linear, saturated and un-substituted, preferably are O-C₂H₅.

In yet another preferred embodiment, the benzotriazole derivatives of formula I according to the invention are compounds wherein
R₅, R₆ and R₇ are identical or different from each other and represent a group of general formula (II) wherein
m=1;
p=0;
R₈, R₉ and R₁₂ are identical or different from each other and represent an optionally substituted (C₁-C₄)alkyl radical.

More preferably R₅, R₆ and R₇ are O-Si(CH₃)₃.

In a preferred embodiment, the benzotriazole derivatives with general formula I according to the invention are compounds wherein

R₁ and R₂ independently from another are selected from H, OH, F, Cl; (C₁-C₄)alkyl, branched or linear, saturated or unsaturated, substituted or un-substituted; O-(C₁-C₄)alkyl, branched or linear, saturated or unsaturated, substituted or un-substituted; preferably R₁ and R₂ are selected from H.

In a preferred embodiment, the benzotriazole derivatives according general formula I according to the invention are compounds wherein
R₃ and R₄ independently from another are selected from H, OH, F, Cl; (C₁-C₄)alkyl, branched or linear, saturated or unsaturated, substituted or un-substituted; O-(C₁-C₄)alkyl, branched or linear, saturated or unsaturated, substituted or un-substituted, preferably R₃ and R₄ are selected from H.

In a preferred embodiment the benzotriazole derivatives according general formula I according to the invention are compounds wherein X is selected from a connecting alkyl-group with 2, 3 or 4 carbon atoms, branched or linear, saturated or unsaturated, substituted or un-substituted; preferably X is a connecting alkyl-group with 2, 3 or 4 carbon atoms, branched or linear, saturated and un-substituted; most preferably X is a linear connecting alkyl-group with 2, 3 or 4, most preferably 3 carbon atoms.

In a preferred embodiment the benzotriazole derivative according general formula I according to the invention is selected from the group consisting of:
5-[3-(triethoxysilyl)propoxy]-2-(2H-benzo[d][1,2,3]triazol-2-yl)benzene-1,3-diol;
2-(2H-benzo[d] [1,2,3]triazol-2-yl)-5-(3-(3,3,3-trimethyl-1,1-bis((trimethylsilyl)oxy)disiloxanyl)propoxybenzene-1,3-diol;
optionally in the form shown or in form of a salt, especially a physiologically, dermatologically or cosmetically acceptable salt, or in form of a solvate, especially a hydrate.

In a very preferred embodiment, the benzotriazole derivatives of general formula I 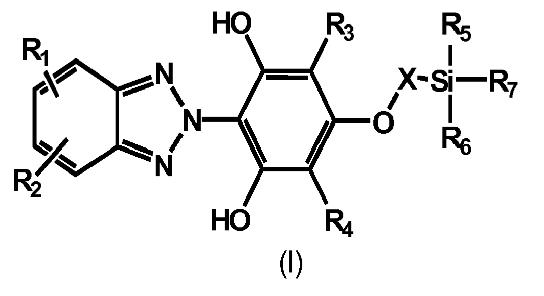 according to the invention are compounds wherein
R₁ and R₂ independently from another are selected from H, OH, F, Cl; (C₁-C₄)alkyl, branched or linear, saturated or unsaturated, substituted or un-substituted; O-(C₁-C₄)alkyl, branched or linear, saturated or unsaturated, substituted or un-substituted; preferably R₁ and R₂ are selected from H;
R₃ and R₄ independently from another are selected from H, OH, F, Cl; (C₁-C₄)alkyl, branched or linear, saturated or unsaturated, substituted or un-substituted; O-(C₁-C₄)alkyl, branched or linear, saturated or unsaturated, substituted or un-substituted, preferably R₃ and R₄ are selected from H;
R₅, R₆ and R₇ independently from another are selected from H; (C₁-C₄)alkyl or O-(Cᵢ-C₄)alkyl, branched or linear, saturated or unsaturated, substituted or unsubstituted, preferably are O-(C₁-C₄)alkyl, branched or linear, saturated and un-substituted, most preferably are O-C₂H₅;
   or
one or two of the radicals R₅, R₆ or R₇ independently from another represent (C₁-C₄)alkyl, branched or linear, saturated or non-saturated, substituted or un-substituted, while the remaining two or one of the radicals R₅, R₆ or R₇ represent a group of general formula (II), preferably one of the radicals R₅, R₆ or R₇ represents (C₁-C₄)alkyl, branched or linear, saturated or non-saturated, substituted or un-substituted, while the remaining two radicals R₅, R₆ or R₇ represent a group of general formula (II) 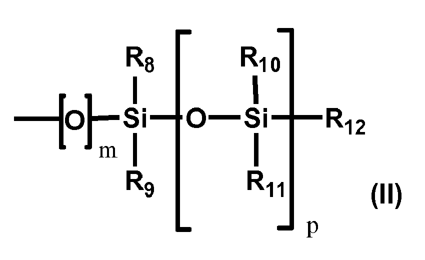 in which
   m= 1;
   p= 0, 1, 2, 3, or 4;
   R₈, Rg, R₁₀, R₁₁ and R₁₂ are identical or different from each other and represent an optionally substituted (C₁-C₆)alkyl radical; an O-(C₁-C₆)alkyl radical; or an -OSi(R₁₃)₃ radical;
R₁₃ represents an (C₁-C₆)alkyl radical, or an O-(C₁-C₆)alkyl radical;
   or
R₅, R₆ and R₇ are identical or different from each other and represent a group of general formula (II) wherein
   m=1,
   p=0,
   R₈, R₉ and R₁₂ are identical or different from each other and represent an optionally substituted (C₁-C₄)alkyl radical; more preferably R₅, R₆ and R₇ are O-Si(CH₃)₃;
X is selected from a connecting alkyl-group with 2, 3 or 4 carbon atoms, branched or linear, saturated or unsaturated, substituted or un-substituted; preferably X is a connecting alkyl-group with 2, 3 or 4 carbon atoms, branched or linear, saturated and un-substituted; most preferably X is a linear connecting alkyl-group with 2, 3 or 4, most preferably 3 carbon atoms;
optionally in the form of any of its racemates or tautomers; in the form shown or in form of a salt, especially a physiologically, dermatologically or cosmetically acceptable salt, or in form of a solvate, especially a hydrate.

Another aspect the present invention provides a process for the production of a benzotriazole derivative according general formula I according to the invention, in which
a) a compound of formula III, wherein R₃ and R₄ are defined as above 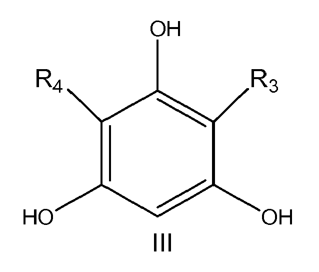 is reacted with benzenesulfonyl chloride to yield a compound of formula IV 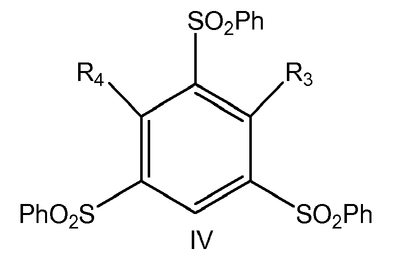
b) following that the compound according to formula IV is reacted with KOH to yield a compound of formula V; 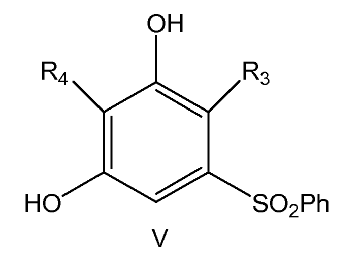
c) following that the compound according to formula V is reacted with K₂CO₃ and 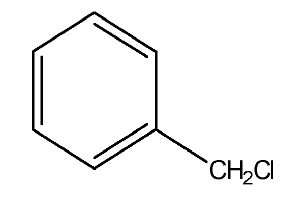 to yield a compound of formula VI; 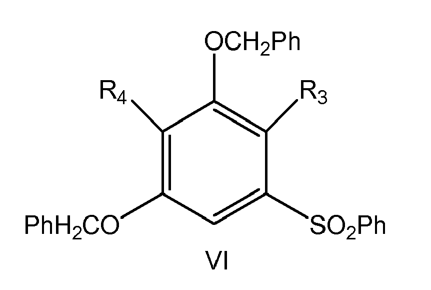
d) following that the compound according to formula VI is reacted with KOH to yield a compound of formula VII 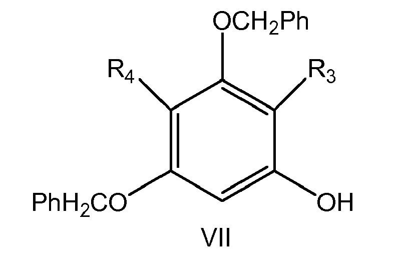
e) following that the compound according to formula VII is reacted with a compound according to formula VIII, wherein R₁ and R₂ are defined as above, which is before activated by NaNO₂ and HCl 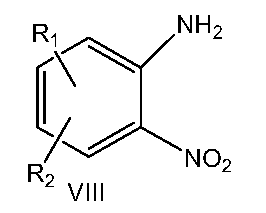 to yield a compound according to formula IX; 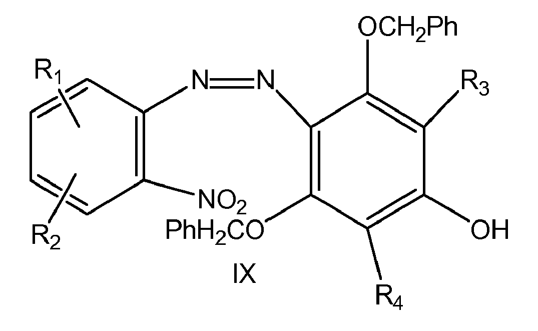
f) following that the compound according to formula IX is reacted with 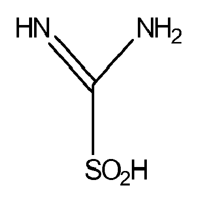 to yield a compound of formula XI 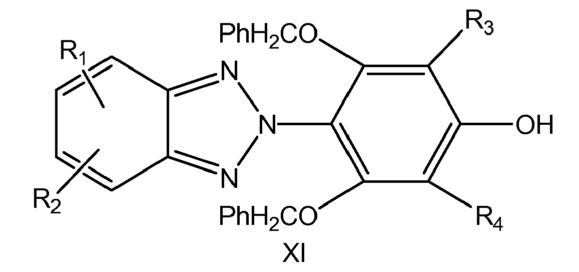
g) following that the compound according to formula XI is reacted first with NaH and then with 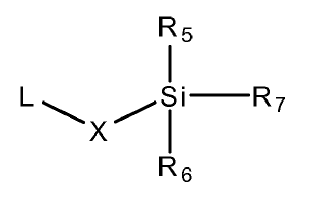 wherein R₅, R₆, X and R₇ are defined as above, and L is a leaving group, and preferably represents chlorine, bromine, iodine or another well known leaving group for those who are skilled in the art, together with KI to yield a compound according to formula XIII 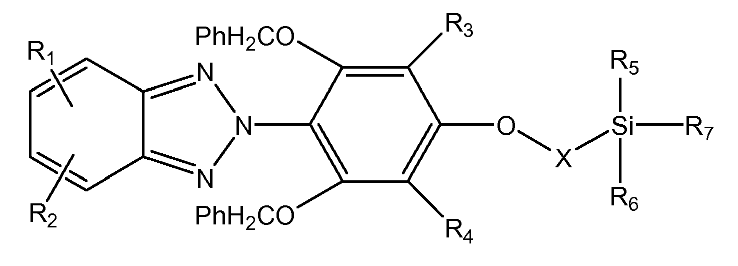
h) following that the compound according to formula XIII is reacted Pd/C and Cyclohexene to yield the compound according to formula I.

It is to be understood that the end-product and all the intermediates may but do not have to be purified, especially by chromatography.

The purification and isolation of the inventive compounds of general formula I may, if required, be carried out by conventional methods known to those skilled in the art, e.g. chromatographic methods or recrystallization.

The compounds of general formula I given above, their stereoisomers, corresponding salts thereof and corresponding solvates are toxicologically acceptable and are therefore suitable as pharmaceutical active substances for the preparation of medicaments.

Thus, another aspect of the present invention relates to a medicament comprising at least one benzotriazole derivative according to the invention, and optionally one or more pharmaceutically acceptable excipients.

The medicament according to the present invention is preferably in any form suitable for the topical application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the use. Conventional pharmaceutical excipients may include inter alia stabilizing agents or solubilizing agents.

Another preferred aspect of the invention refers to dermatological, cosmetic, veterinarian or pharmaceutical formulation comprising at least one benzotriazole derivative according to the invention, and optionally one or more dermatologically acceptable excipients.

Another preferred aspect of the invention refers to a dermatological formulation comprising at least one benzotriazole derivative according to the invention, and optionally one or more dermatologically acceptable excipients.

Another preferred aspect of the invention refers to a cosmetic formulation, comprising at least one benzotriazole derivative according to the invention, and optionally one or more cosmetically acceptable excipients.

Another preferred aspect of the invention refers to a veterinarian formulation comprising at least one benzotriazole derivative according to the invention, and optionally one or more pharmaceutically acceptable excipients.
Another preferred aspect of the invention refers to a pharmaceutical formulation comprising at least one benzotriazole derivative according to the invention, and optionally one or more pharmaceutically acceptable excipients.

In a preferred embodiment of any of the medicament, dermatological, cosmetic, veterinarian or pharmaceutical formulation according to the invention the medicament, dermatological, cosmetic, veterinarian or pharmaceutical formulation is in the form of a crème, especially a suncreme; a shampoo, a gel, a lacquer, a foam, a lotion, a soap, an ointment an emulsion a suspension or a solution.

In a preferred embodiment of any of the medicament, dermatological, cosmetic, veterinarian or pharmaceutical formulation according to the invention the medicament, dermatological, cosmetic, veterinarian or pharmaceutical formulation comprises additionally at least one filter agent against solar radiation, preferably UV, which preferably may be organic, inorganic, or micronized.

In a preferred embodiment of any of the medicament, dermatological, cosmetic, veterinarian or pharmaceutical formulation according to the invention the benzotriazole derivative according to the invention is micronized.

Said cosmetic, dermatological or pharmaceutical formulation can be adapted for application thereof on the skin and lips in the form of: a non-ionic vesicular dispersion, emulsion, cream, lotion, gel, aerosol, cream-gel, gel-cream, suspension, dispersion, ointment, powder, solid stick, foam, spray, oil, pomade and fluid, among others.

Similarly, said formulation can be adapted for applying it on the hair in the form of a shampoo, lotion, gel, fluid, lacquer, foam, dye, emulsion, cream, spray, among others, and on the nails in the form of a nail varnish, oil and gel, among others.

The organic, micronized organic and inorganic filters are selected from those acceptable under the country's legislation.

The organic filters, for example, can be selected from those approved by the Council of the European Communities (revised text of European Directive 76/768/EEC Annex-7, pages 76-81, published on 15.10.2003) and by the U.S. Food and Drug Administration (see, for example, "Food and Drugs, Sunscreen drug products for over the counter human use", title 21, volume 5 of Code of Federal Regulations, revised 1 April 2004), such as: anthranilates; camphor derivatives; dibenzoylmethane derivatives; benzotriazole derivatives; diphenylacrylate derivatives; cinnamic derivatives; salycylic derivatives; triazine derivatives such as those disclosed in patents EP-863145, EP-517104, EP-570838, EP-796851, EP-775698 and EP-878469, benzophenone derivatives; benzalmalonate derivatives; benzimidazole derivatives, imidizolines; p-aminobenzoic acid derivatives; polymeric and silicone filters.

The inorganic filters can be selected from a group that includes: metallic oxides as pigments, nanopigments, treated and untreated, such as the dioxide of titanium (amorphous or crystalline), iron, zinc, zirconium or cerium. Moreover, alumina and/or aluminium stearate are conventional coating agents, while examples of untreated metallic oxides as (uncoated) inorganic filters are those described in patents EP518772 and EP518773.

The cosmetic, dermatological and pharmaceutical formulations of the present invention can additionally contain additives and adjuvants that can be selected from fatty acids, organic solvents, thickening agents, softening agents, antioxidants, opacifiers, stabilisers, emollients, hydroxyacids, antifoaming agents, moisturizing agents, vitamins, fragrances, preservatives, surfactants, sequestering agents, polymers, propellants, acidifying or basifying agents, colorants, dyes, dihydroxyacetone, insect repellent or any other ingredient that is commonly used in cosmetic formulations, and particularly in the production of photo-protective compositions.

Examples of substances/fatty acids include, among others, oils or waxes or mixtures thereof and can include fatty acids, fatty alcohols and fatty acid esters. The oils are advantageously selected from animal and vegetable oils, mineral or synthetic oils, and in particular, from liquid petrolatum, liquid paraffin, volatile and non volatile silicone oils, isoparaffins, polyalphaolefins or fluorated or perfluorated oils. Similarly, the waxes are advantageously selected from animal and vegetable waxes, mineral or synthetic waxes known to skilled in the art.

Examples of organic solvents include short alcohols and polyols.

The thickeners are selected, advantageously, from among acrylic-acid cross-linked polymers, modified and unmodified carob bean rubbers, celluloses and xanthane rubbers, such as hydroxypropylated carob bean rubber, methylhydroxyethylcellulose, hydroxypropylmethylcellulose or hydroxyethylcellulose.

When choosing the excipients, adjuvants, etc., a person skilled in the art will ensure that they do not affect the activity of the benzotriazole derivatives of general formula (I) in accordance with the invention.

Another preferred aspect of the invention refers to the use of a benzotriazole derivative according to the invention as UV-filter agent.

Another preferred aspect of the invention refers to the use of a benzotriazole derivative according to the invention for the preparation of a medicament, or a dermatological, cosmetic, veterinarian or pharmaceutical formulation for the protection of the skin, the lips, the hair or the nails against damage from solar radiation, preferably UV.

Another preferred aspect of the invention refers to the use of a benzotriazole derivative according to the invention for the preparation of a medicament, or a dermatological, cosmetic, veterinarian or pharmaceutical formulation for the prevention, the coadjuvant therapy or the treatment of diseases caused by solar radiation, especially UV-radiation.

Another preferred aspect of the invention refers to the use of a benzotriazole derivative according to the invention as photostabilizer, especially in polymers.

In the present invention "polymer" is understood as chemical compounds of natural or synthetic origin and generally of higher molecular weight formed from single structural units (monomers) which are linked to each other through covalent bonds. Examples of polymers include but are not limited to, proteins, polysaccharides, cellulose, gum arabic, nucleic acids, polyethylene, polycarbonates, polymers of silicone, polyurethanes, polyesters, polyamides and acrylic polymers.

Another preferred aspect of the invention refers to the use of a benzotriazole derivative according to the invention as UV filter agent in textile fibers.

Another preferred aspect of the invention refers to the use of a benzotriazole derivative according to the invention as photo-stabilizer, especially in polymers; or as UV filter agent in textile fibers.

The present invention is illustrated below with the aid of examples. These illustrations are given solely by way of example and do not limit the general spirit of the present invention.

### Examples and Figures

Figure1 shows the UV spectrum of example 1 measured by methods well known in the art.

Figure 2 shows the UV spectrum of compound 14 (example 2) measured by methods well known in the art.

The following compounds were prepared according to the general processes described above. Those skilled in the art are familiar with the starting materials that are needed to obtain said compounds.

### Example 1: Preparation of 5-[3-(triethoxysilyl)propoxy]-2-(2H-benzo[d][1,2,3]triazol-2-yl)benzene-1,3-diol (1)

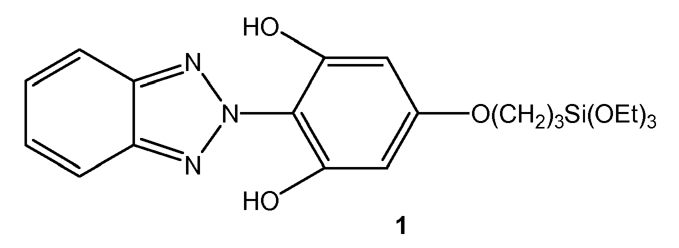

Phloroglucinol tribenzenesulfonate (**3**) was prepared from phloroglucinol (**2**; yield 93 %) according to the method of E.M. Kampouris (J. Chem. Soc. 1965, 2651).

3,5-Dihydroxyphenyl benzylsulfonate (**4**) and 3,5-bis(benzyloxy)phenyl benzenesulfonate (**5**) were prepared by the method of G. Büchi at al (J. Am. Chem. Soc. 1981, 103, 3499. Yields 54% and 94%, respectively).

3,5-bis(Benzyloxy)phenol (**6**) was prepared according to the method described by G. Büchi at al (J. Am. Chem. Soc. 1981, 103,3497. Yield 84.6%).

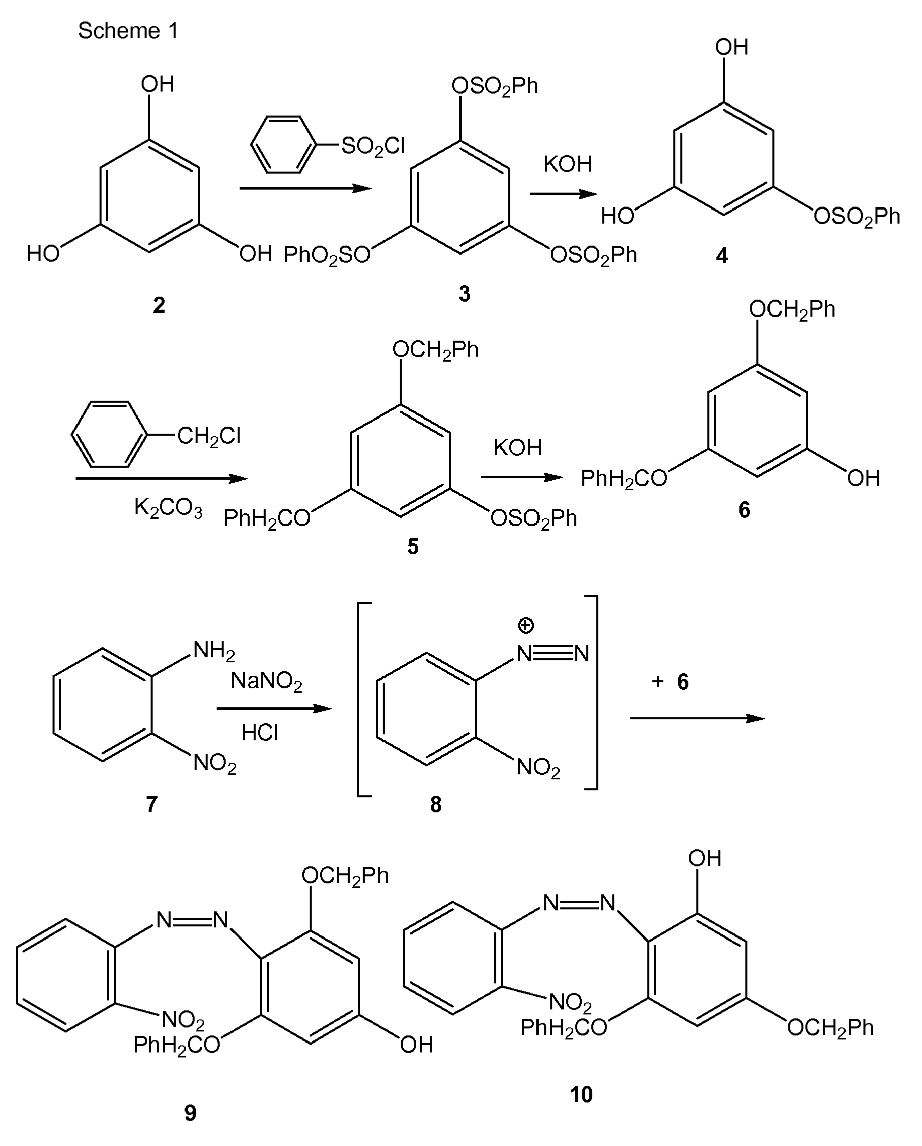

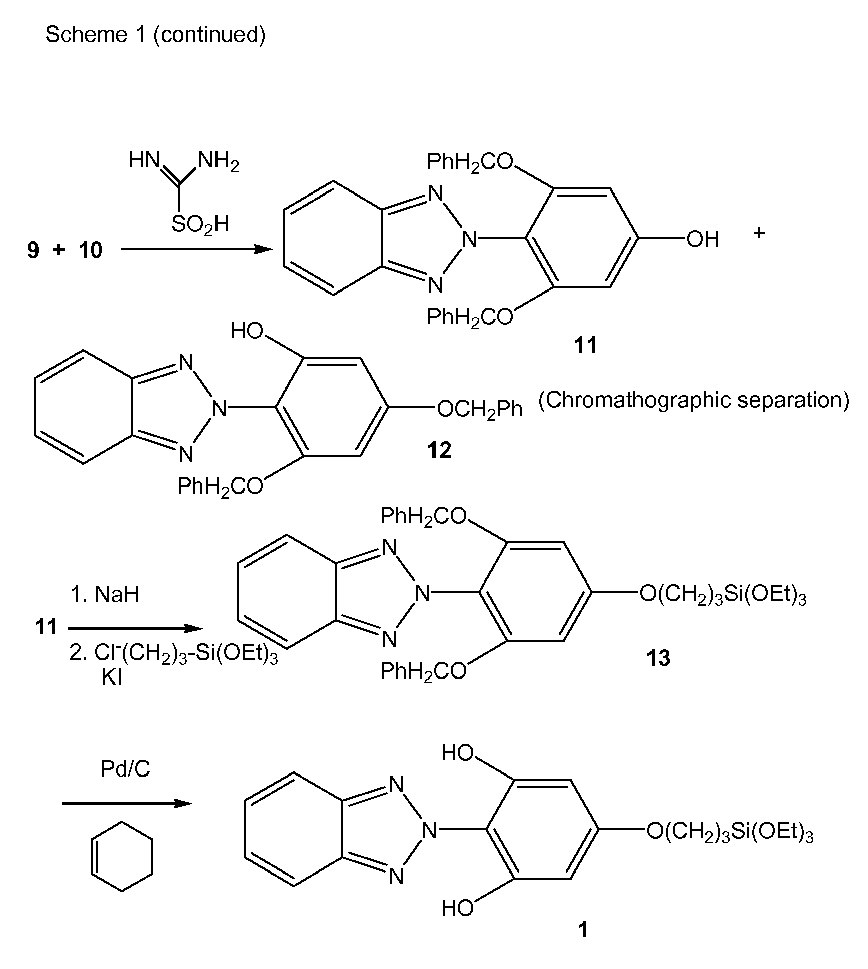

### 4-(2H-benzo[d][1,2,3]triazol-2-yl)-3,5-bis(benzyloxy)phenol (11)

To a cold (0-5°) suspension of 2-nitrobenzeneamine (7; 27.62 g, 0.2 mol) in concentrated HCl (103 ml), crashed ice (100 g) and water (100 ml), was added dropwise 5N solution of NaNO₂ (41.2 ml). The resulting mixture was stirred at 5° for 1 h and then filtered. This solution was then added dropwise to a solution of 3,5-bis(benzyloxy)phenol (**6**; 61.27 g, 0.2 mol) in MeOH (600 ml) with stirring. The reaction mixture was stirred at r.t. for 2 h and the precipitated red crystalline compound was collected by filtration to give a mixture of two isomeric azo-compounds (**9** and **10**).
Yield: 80.77 g (88%). Mp 171-180°C. R_{f} = 0.52 and 0.88 (toluene-MeOH 9:1).

To a boiling suspension of the above mixture of azo-compounds (45.5 g; 0.1 mol) in a mixture of EtOH (250 ml) and 4N NaOH (250 ml) was added formamidinesulfinic acid (23.65 g; 0.22 mol) during 0.5 h. The resulting mixture was heated with stirring for 1 h, cooled to r.t., and then poured into a mixture of ice and water (700 g). The mixture was acidified with concentrated HCl (80 ml) and extracted with EtOAc. The organic layer was successively washed with water and brine, and then dried (MgSO₄). Evaporation of the solvent in vacuo afforded a red oil (42 g), which was then treated with a mixture of hexane and EtOAc (60 ml, each). The precipitated crystalline compounds were collected by filtration and washed with hexane-acetone (1:1).
Yield: 12.64 g (28%). Mp 147-152°C. R_{f} = 0.38 (toluene-MeOH 9:1), = 0.26 (hexane-EtOAc 2:1).
¹H-NMR(CDCl₃): 4.73 (s, 4H, 2 OCH₂), 6.11 (s, 2H, C₂-H and C₆-H), 6.98 (m, 4H, arom.
H-s), 7.07 (m, 2H, arom. H-s), 7.10 (m, 4H, arom. H-s), 7.43 (dd, J=6.7 and 3 Hz, 2H, C_{5'}-H and C_{6'}-H), 7.95 (dd, J=6.7 and 3 Hz, 2H, C_{4'}-H and C_{7'}-H), 8.62 (br.s, 1H, OH).
¹³C-NMR(CDCl₃): 70.31 (OCH₂), 94.55 (C-2 and C-6), 113.03 (C-4), 118.23 (C-4' and C-7'), 126.61, 126.88 (C-5' and C-6'), 127.65, 128.27, 136.01, 144.37 (C-3a' and C-7a'), 156.00 (C-3 and C-5), 159.99 (C-1).

The isomer **12**: Mp 71-78 °C. R_{f}= 0.73 (toluene-MeOH 9:1), 0.48 (hexane-EtOAc 2:1 )

¹H-NMR(CDCl₃): 5.04 (s, 2H, 2 OCH₂), 5.16 (s, 2H, OCH₂), 6.38 (d, J= 2.4 Hz, 1 H, C₄-H), 6.41 (d, J= 2.4 Hz, 1 H, C₆-H), 7.25 (t, J= 7 Hz, 1 H, arom.H), 7.30 (t, J= 7.5 Hz, 2H, arom. H), 7.35 (t, J= 7.2 Hz, 1 H, arom. H), 7.39 (m, 4H, arom.H), 7.45 (dd, *J*=6.5 Hz, 2H, C_{5'}-H and C₆,-H), 7.94 (dd, J= 6.5 Hz, 2H, C₄,-H and C₇,-H), 9.36 (br.s, 1 H, OH).
¹³C-NMR(CDCl₃): 70.33 (OCH₂), 71.28 (OCH₂), 95.21 (C-4), 95.81 (C-6), 112.50 (C-2), 117.87 (C-4' and C-7'), 126.86, 127.23 C-5' and C-6'), 127.62, 128.24, 128.41, 128.68, 136.18, 136.30 , 143.64 (C-3a' and C-7a'), 152.80, 153.67 (C-3), 160.62 (C-5).

### 2-[4-(3-(Triethoxysilyl)propoxy]-2,6-bis(benzyloxy)phenyl]-2H-benzo[d][1,2,3]triazole (13)

To a stirred suspension of NaH (1.8 g, 60% in mineral oil, 44 mmol) in dry DMF (80 ml) was added dropwise a solution of compound 11 (16.9 g, 40 mmol) in dry DMF (140 ml). After stirring for 45 min at r.t. chloropropyl-triethoxy-silane (9.87 g, 40 mmol) and Kl (6.43 g, 40 mmol) were added, and the resultant mixture was heated at 80 °C for 8 h. After cooling, the reaction mixture was poured into water (200 ml), extracted with ether (tree times, 150 ml each), the organic layer was washed with brine, and then dried (MgSO₄). Evaporation of the solvent in vacuo afforded a redish oil, which was purified by column chromatography (hexane-EtOAc 5:1 as eluent).
Yield: 9.13 g (37%), semi solid. R_{f}= 0.7 (hexane-EtOAc 2:1).
¹H-NMR (CDCl₃): 0.79 (m, 2H, CH₂-Si), 1.27 (t, J=7 Hz, 9H, 3 CH₃), 1.93 (m, 2H, CH₂), 3.88 (q, J= 7 Hz,, 6H, 3 OCH₂), 3.92 (t, J= 6.6 Hz, 2H, OCH₂), 5.07 (s, 4H, 2 CH₂), 6.28 (s, 2H, C_{3'}-H and C_{5'}-H), 7.17 (d, J= 7.5 Hz, 4H, arom.H), 7.23 (m, 6H, arom.H), 7.44 (dd, J= 6.6 and 3 Hz, 2H, C₅-H and C₆-H), 8.1 (dd, J= 6.6 and 3 Hz, 2H, C₄-H and C₇-H).
¹³C-NMR (CDCl₃): 6.47 (C-Si), 18.30 (CH₃), 22.62 (C-C-Si), 58.43 (C-0),70.16 (C-O), 70.57 (C-O), 93.38 (C-3' and C-5'), 114.53 (C-1'), 118.47 (C-4 and C-7), 126.29 (C-5 and C-6), 126.52 (arom.C), 127.62 (arom.C), 128.32 (arom.C), 136.19 (arom.C), 144.74 (C-3a and C-7a), 156.06 (C-2' and C-6'), 161.65 (C-4').

### 5-[3-(Triethoxysilyl)propoxy]-2-(2H-benzo[d][1,2,3]triazol-2-yl)benzene-1,3-diol (1)

A stirred mixture of compound **13** (2 g, 3.2 mmol), palladium catalyst (1 g, 10% Pd on charcoal), and cyclohexene (64 ml, 52 g, 0.6 mol) in dry EtOH (80 ml) was heated at reflux for 1.5 h. After filtration, the solvent was evaporated in vacuo and the residue was purified by column chromatography to yield 1.08 g (76 %), light yellow crystals. Mp 69-74 °C. R_{f}= 0.27 (CH₂Cl₂).
**¹H NMR (CDCl₃):** 0.80 (m, 2H, CH₂-Si), 1.27 (t, J= 7 Hz, 9H, 3 CH₃), 1.95 (m, 2H, CH₂), 3.88 (q, J= 7 Hz, 6H, 3 O-CH₂), 4.00 (t, J= 6.6 Hz, 2H, CH₂), 6.29 (s, 2H, C₅-H and C₆-H), 7.93 (m, 2H, C₄-H and C₇-H), 11.56 (s, 2H, OH).
**¹³C NMR (CDCl₃):** 6.45 (C-Si), 18.29 (CH₃), 22.64 (C-2 "), 58.43 (C-O), 70.10 (C-1 "), 95.73 (C-6), 108.17 (C-2), 116.62 (C-4' and C-7'), 127.87 (C-5' and C-6'), 140.18 (C-3a and C-7a), 152.07 (C-1 and C-3), 160.93 (C-5).

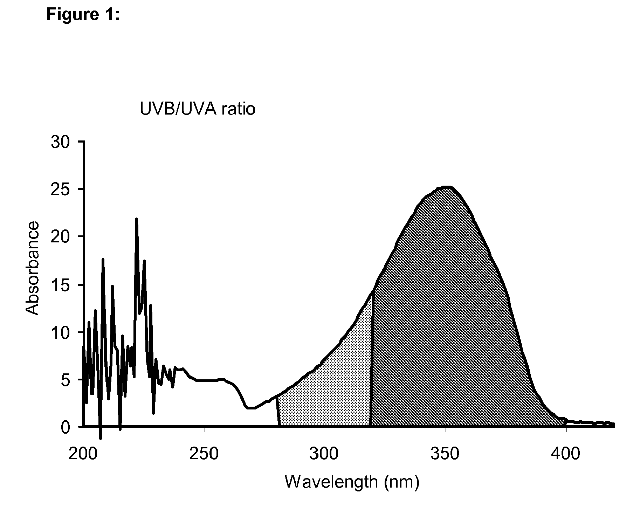

**UV-Filter-Spectrum:** A UV-Spectrum of the compounds was measured according to standard methods known in the art. The spectrum of the compound according to example 1 was taken (see Figure 1) and its absorption factor εₘₒₗₐᵣ and its λₘₐₓ determined. εₘₒₗₐᵣ was 25354 M⁻¹ cm⁻¹ (CHCl₃) with 81 % of its absorption being in the UVA range. λₘₐₓ was determined at 350nm.

### Example 2: Preparation of 2-(2H-benzo[d][1,2,3]triazol-2-yl)-5-(3-(3,3,3-trimethyl-1,1-bis((trimethylsilyl)oxy)disiloxanyl)propoxybenzene-1,3-diol (14)

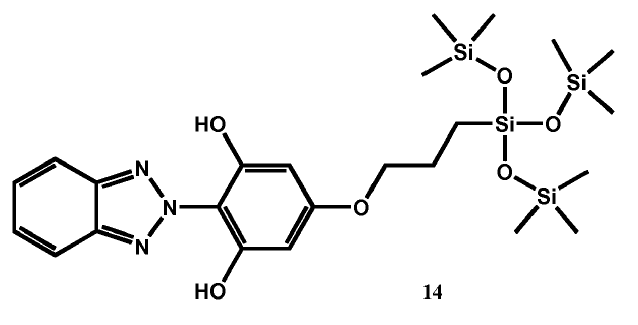

Compound **14** was prepared according the procedure described in example 1 starting from 4-(2H-benzo[d][1,2,3]triazol-2-yl)-3,5-bis(benzyloxy)phenol (11) and chloropropyl-[tris(trimethylsilyloxy)]-silane.

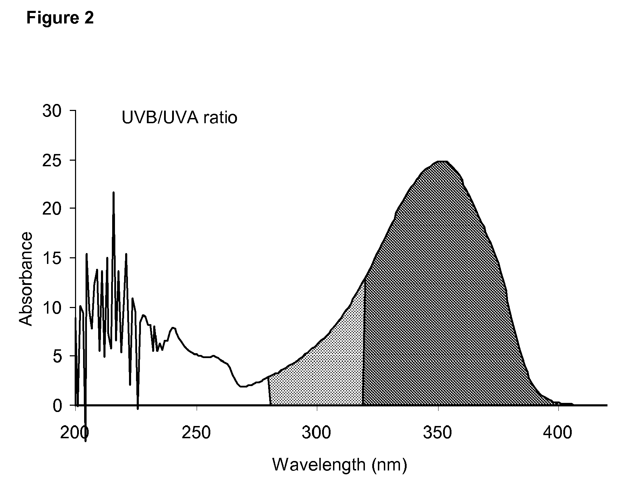

**¹H NMR (CDCl₃):** 0.14 (s, 27H, 9 CH₃), 0.62 (m,2H, CH₂), 1.86 (m, 2H, CH₂), 3.98 (t, J=6.7 Hz, 2H, O CH₂), 6.31 (s, 2H, C₄-H and C₆-H), 7.55 (dd, J =6.6 and 3.0 Hz, 2H, C₅-H and C_{6'}-H), 7.94 (dd, J=6.6 and 3.0 Hz, 2H, C_{4'}-H and C_{7'}-H), 11.56 (s, 2H, 2 OH).
**¹H NMR (CDCl₃):** 1.74 (CH₃), 10.39 (C-3"), 23.18 (C-2"), 70.37 (C-1"), 95.76 (C-4 and C-6), 108.16 (C-2), 116.63 (C-4' and C-7'), 127.87 (C-5' and C-6'), 140.21 (C-3a' and C-7a'), 152.09 (C-1 and C-3), 161.03 (C-5).
UV-Filter-Spectrum: A UV-Spectrum of the compound was measured according to standard methods known in the art. The spectrum of the compound according to example 2 was taken (see Figure 2) and its absorption factor εmolar and its λₘₐₓ determined. εmolar was 24879 M⁻¹ cm⁻¹ (CHCl₃) with 83 % of its absorption being in the UVA range. λₘₐₓ was determined at 372nm.

## Claims

**1.** Benzotriazole derivatives of general formula I, 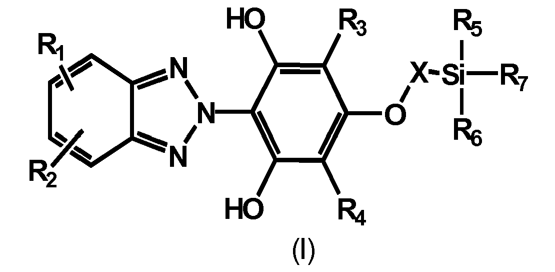 wherein
R₁ and R₂ independently from another are selected from H, OH, SH, F, Cl, Br, I, NH₂, (C₁-C₁₀)alkyl optionally substituted, and O-(C₁-C₁₀)alkyl optionally substituted;
R₃ and R₄ independently from another are selected from H, OH, SH, F, Cl, Br, I, NH₂, (C₁-C₁₀)alkyl optionally substituted, and O-(C₁-C₁₀)alkyl optionally substituted;
R₅, R₆, R₇ independently from another are selected from H, (C₁-C₄)alkyl optionally substituted, O-(C₁-C₄)alkyl optionally substituted, an (C₅-C₂₀)aryl group optionally substituted, O-(C₅-C₂₀)aryl group optionally substituted, (C₁-C₄)alkyl-(C₅-C₂₀)aryl optionally substituted, O-(C₁-C₄)alkyl-(C₅-C₂₀)aryl optionally substituted, or a group of general formula (II) 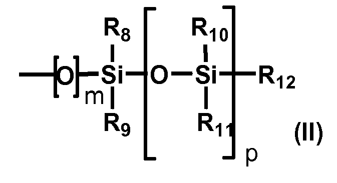 in which
m= 0 or 1;
p= 0, 1, 2, 3, or 4;
R₈, Rg, R₁₀, R₁₁ and R₁₂ are identical or different from each other and represent an optionally substituted (C₁-C₆)alkyl, an O-(C₁-C₆)alkyl radical, an optionally substituted (C₅-C₂₀)aryl radical or an -OSi(R₁₃)₃ radical;
R₁₃ represents an (C₁-C₆)alkyl radical, an O-(C₁-C₆)alkyl radical or an optionally substituted (C₅-C₂₀)aryl radical;
X is a connecting alkyl-group with 1, 2, 3, 4, 5 or 6 carbon atoms; branched or linear, saturated or unsaturated, substituted or un-substituted;
wherein the substituents are selected from the group consisting of F, Cl, Br, I, NH₂, SH and OH;
optionally in the form of any of its racemates or tautomers; in the form shown or in form of a salt, especially a physiologically, dermatologically or cosmetically acceptable salt, or in form of a solvate, especially a hydrate.

**2.** Benzotriazole derivatives of general formula according to claim 1, 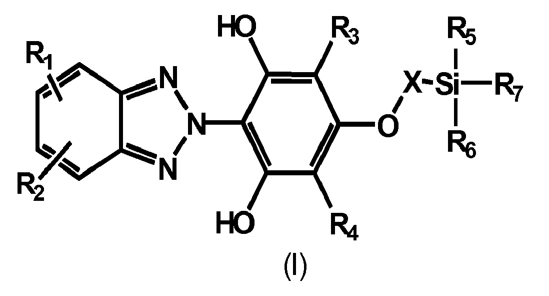 wherein
R¹ and R² independently from another are selected from H, OH, SH, F, Cl, Br, I, NH₂; (C₁-C₁₀)alkyl, branched or linear, saturated or unsaturated, substituted or unsubstituted; O-(C₁-C₁₀)alkyl, branched or linear, saturated or unsaturated, substituted or unsubstituted;
R³ and R⁴ independently from another are selected from H, OH, SH, F, Cl, Br, I, NH₂; (C₁-C₁₀)alkyl, branched or linear, saturated or unsaturated, substituted or unsubstituted; O-(C₁-C₁₀)alkyl, branched or linear, saturated or unsaturated, substituted or un-substituted;
R⁵, R⁶, R⁷ independently from one another are selected from H; (C₁-C₄)alkyl optionally substituted; O-(C₁-C₄)alkyl optionally substituted; an (C₅-C₂₀)aryl group optionally substituted; O-(C₅-C₂₀)aryl group optionally substituted; (C₁-C₄)alkyl-(C₅-C₂₀)aryl optionally substituted; or a group of general formula (II) 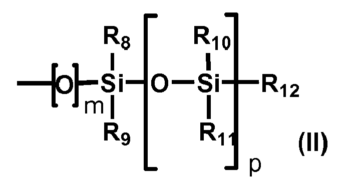 in which
m= 1;
p= 0, 1, 2, 3, or 4;
R₈, Rg, R₁₀, R₁₁ and R₁₂ are identical or different from each other and represent an optionally substituted (C₁-C₆)alkyl, an O-(C₁-C₆)alkyl radical, or an -OSi(R₁₃)₃ radical;
R₁₃ represents an (C₁-C₆)alkyl radical, or an O-(C₁-C₆)alkyl radical;
X is a connecting alkyl-group with 1, 2, 3, 4, 5 or 6 carbon atoms; branched or linear, saturated or unsaturated, substituted or un-substituted;
optionally in the form of any of its racemates or tautomers; in the form shown or in form of a salt, especially a physiologically, dermatologically or cosmetically acceptable salt, or in form of a solvate, especially a hydrate.

**3.** Benzotriazole derivatives according to any of claims 1 or 2, wherein
one or two of the radicals R₅, R₆ or R₇ represent (C₁-C₄)alkyl optionally substituted, while the remaining two or one of the radicals R₅, R₆ or R₇ represent a group of general formula (II) as defined in claim 1 or 2;
or alternatively
R₅, R₆ and R₇ independently from another are selected from H; (C₁-C₄)alkyl optionally substituted or O-(C₁-C₄)alkyl optionally substituted;
or alternatively
R₅, R₆ and R₇ are identical or different from each other and represent a group of general formula (II) wherein
m=1;
p=0;
R₈, R₉ and R₁₂ are identical or different from each other and represent an optionally substituted (C₁-C₄)alkyl radical.

**4.** Benzotriazole derivatives according to any of claims 1 to 3, wherein
R₁ and R₂ independently from another are selected from H, OH, F, Cl, (C₁-C₄)alkyl optionally substituted, and O-(C₁-C₄)alkyl optionally substituted.

**5.** Benzotriazole derivatives according to any of claims 1 to 4, wherein
R₃ and R₄ independently from another are selected from H, OH, F, Cl, (C₁-C₄)alkyl optionally substituted, and O-(C₁-C₄)alkyl optionally substituted.

**6.** Benzotriazole derivatives according to any of claims 1 to 5, wherein X is selected from a connecting alkyl-group with 2, 3 or 4 carbon atoms, branched or linear, saturated or unsaturated, substituted or un-substituted.

**7.** Benzotriazole derivatives according to any of claims 1 to 2, wherein the derivative is selected from the group consisting of:
5-[3-(triethoxysilyl)propoxy]-2-(2H-benzo[d][1,2,3]triazol-2-yl)benzene-1,3-diol;
2-(2H-benzo[d] [1,2,3]triazol-2-yl)-5-(3-(3,3,3-trimethyl-1,1-bis((trimethylsilyl)oxy)disiloxanyl)propoxybenzene-1,3-diol;
optionally in the form shown or in form of a salt, especially a physiologically, dermatologically or cosmetically acceptable salt, or in form of a solvate, especially a hydrate.

**8.** Benzotriazole derivatives of general formula I according to claim 1, 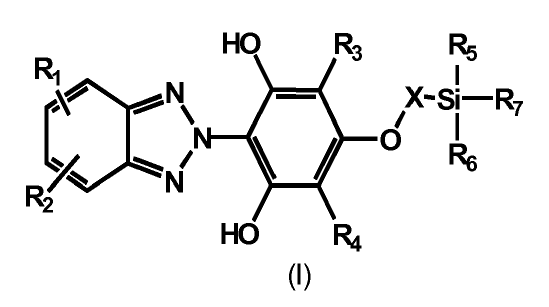 wherein
R₁ and R₂ independently from another are selected from H, OH, F, Cl, (C₁-C₄)alkyl optionally substituted, and O-(C₁-C₄)alkyl optionally substituted;
R₃ and R₄ independently from another are selected from H, OH, F, Cl, (C₁-C₄)alkyl optionally substituted, and O-(C₁-C₄)alkyl optionally substituted;
R₅, R₆ and R₇ independently from another are selected from H, (C₁-C₄)alkyl optionally substituted, and O-(C₁-C₄)alkyl optionally substituted;
or
one or two of the radicals R₅, R₆ or R₇ independently from another represent (C₁-C₄)alkyl optionally substituted, while the remaining two or one of the radicals R₅, R₆ or R₇ represent a group of general formula (II) 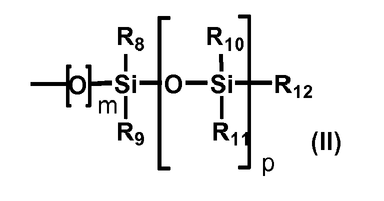 in which
m= 1;
p= 0, 1, 2, 3, or 4;
R₈, Rg, R₁₀, R₁₁ and R₁₂ are identical or different from each other and represent an optionally substituted (C₁-C₆)alkyl radical, an O-(C₁-C₆)alkyl radical, or an -OSi(R₁₃)₃ radical;
or
R₅, R₆ and R₇ are identical or different from each other and represent a group of general formula (II) wherein
m=1;
p=0;
R₈, R₉ and R₁₂ are identical or different from each other and represent an optionally substituted (C₁-C₄)alkyl radical;
R₁₃ represents an (C₁-C₆)alkyl radical, or an O-(C₁-C₆)alkyl radical;
X is selected from a connecting alkyl-group with 2, 3 or 4 carbon atoms, branched or linear, saturated or unsaturated, substituted or un-substituted;
optionally in the form of any of its racemates or tautomers; in the form shown or in form of a salt, especially a physiologically, dermatologically or cosmetically acceptable salt, or in form of a solvate, especially a hydrate.

**9.** Medicament comprising at least one benzotriazole derivative according to any of claims 1 to 8, and optionally one or more pharmaceutically acceptable excipients.

**10.** Dermatological, cosmetic, veterinarian or pharmaceutical formulation comprising at least one benzotriazole derivative according to any of claims 1 to 8, and optionally one or more dermatologically acceptable excipients.

**11.** Any of the medicaments, dermatological, cosmetic, veterinarian or pharmaceutical formulations according to claims 11 or 12 in the form of a crème, especially a suncreme; a shampoo, a gel, a lacquer, a foam, a lotion, a soap, an ointment an emulsion a suspension or a solution.

**12.** Any of the medicament, dermatological, cosmetic, veterinarian or pharmaceutical formulation according to claims 9 to 11 comprising additionally at least one filter agent against solar radiation, preferably UV, which preferably may be organic or inorganic or micronized.

**13.** Any of the medicaments, dermatological, cosmetic, veterinarian or pharmaceutical formulations according to any of claims 9 to 12, in which the benzotriazole derivative according to any of claims 1 to 8 is micronized.

**14.** Use of a benzotriazole derivative according to any of claims 1 to 8 as UV-filter agent.

**15.** Use of a benzotriazole derivative according to any of claims 1 to 8 for the preparation of a medicament, or a dermatological, cosmetic, veterinarian or pharmaceutical formulation for the protection of the skin, the lips, the hair or the nails against damage from solar radiation, preferably UV.

**16.** Use of a benzotriazole derivative according to any of claims 1 to 8 for the preparation of a medicament, or a dermatological, cosmetic, veterinarian or pharmaceutical formulation for the prevention, the coadjuvant therapy or the treatment of diseases caused by solar radiation, especially UV-radiation.

**17.** Use of a benzotriazole derivative according to any of claims 1 to 8 as photo-stabilizer, especially in polymers; or as UV filter agent in textile fibers.

**19.** Process for the production of a benzotriazole derivative according general formula I according to any of claims 1 to 8, in which
a) a compound of formula III, wherein R₃ and R₄ are defined as in claim 1 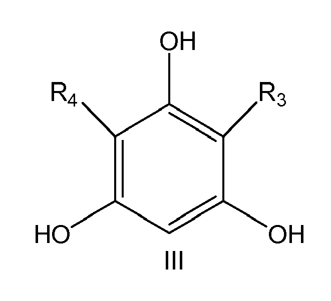 is reacted with benzenesulfonyl chloride, to yield a compound of formula IV 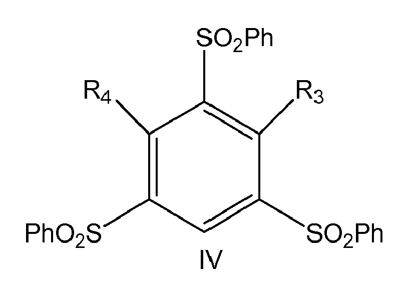
b) following that the compound according to formula IV is reacted with KOH to yield a compound of formula V; 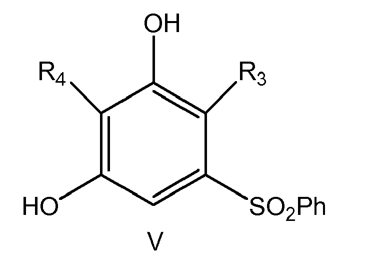
c) following that the compound according to formula V is reacted with K₂CO₃ and 1-(chloromethyl)benzene, to yield a compound of formula VI; 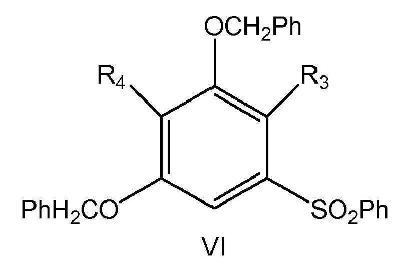
d) following that the compound according to formula VI is reacted with KOH to yield a compound of formula VII 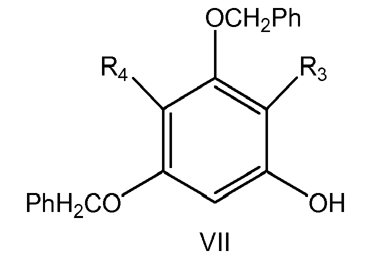
e) following that the compound according to formula VII is reacted with a compound according to formula VIII, wherein R₁ and R₂ are defined as in claim 1, which is before activated by NaNO₂ and HCl 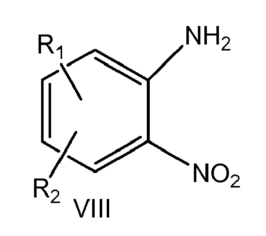 to yield a compound according to formula IX; 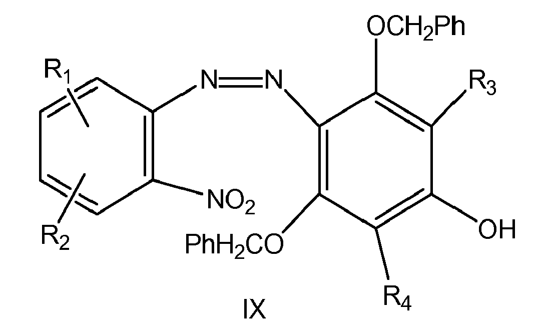
f) following that the compound according to formula IX is reacted with 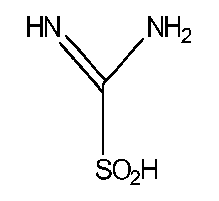 to yield a compound of formula XI 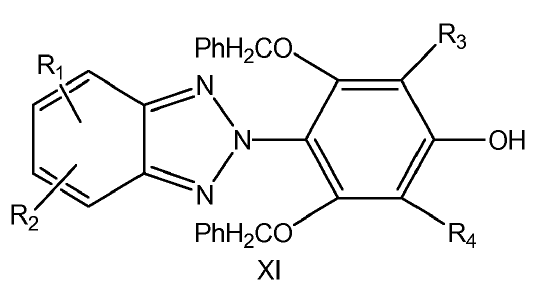
g) following that the compound according to formula XI is reacted first with NaH and then with 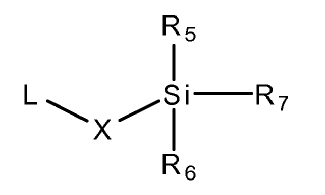 wherein R⁵, R⁶, X and R⁷ are defined as in claim 1, and L is a leaving group, and represents chlorine, bromine, iodine or another leaving group, together with KI to yield a compound according to formula XIII 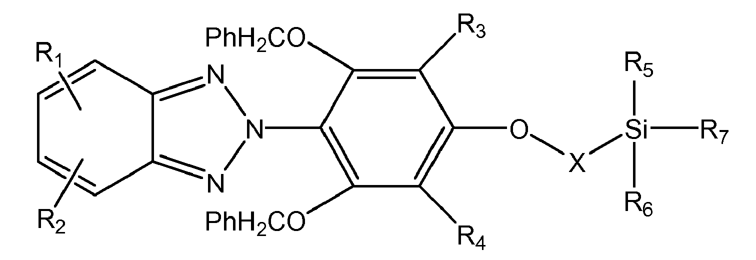
h) following that the compound according to formula XIII is reacted Pd/C and cyclohexene to yield the compound according to formula 1.
